(19) **Europäisches Patentamt**
**European Patent Office**
**Office européen des brevets**

(11) **EP 4 297 633 B1**

(12) **EUROPEAN PATENT SPECIFICATION**

(45) Date of publication and mention
of the grant of the patent:
**22.10.2025 Bulletin 2025/43**

(21) Application number: **22707407.7**

(22) Date of filing: **17.02.2022**

(51) International Patent Classification (IPC):
*A61B 5/00* (2006.01)    *G16H 50/30* (2018.01)

(52) Cooperative Patent Classification (CPC):
**A61B 5/4041; A61B 5/0051; A61B 5/6826;**
**A61B 5/6829; A61B 5/7221; A61B 5/7267;**
**A61B 5/7275; G16H 20/17; G16H 40/63;**
**G16H 50/20; G16H 50/30; G16H 50/70**

(86) International application number:
**PCT/EP2022/054024**

(87) International publication number:
**WO 2022/179938 (01.09.2022 Gazette 2022/35)**

(54) **BIOMARKER FOR PREDICTION OF CHEMOTHERAPY-INDUCED NEUROPATHY**
BIOMARKER ZUR VORHERSAGE VON CHEMOTHERAPIEINDUZIERTER NEUROPATHIE
BIOMARQUEUR DE PRÉDICTION DE LA NEUROPATHIE INDUITE PAR UNE CHIMIOTHÉRAPIE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **26.02.2021 SE 2150214**

(43) Date of publication of application:
**03.01.2024 Bulletin 2024/01**

(73) Proprietor: **Vibrosense Dynamics AB**
**205 12 Malmö (SE)**

(72) Inventors:
• **OLDE, Bo**
**224 67 LUND (SE)**
• **SPEIDEL, Antonio**
**218 71 TYGELSJÖ (SE)**

(74) Representative: **Neij & Lindberg AB**
**Pedellgatan 11**
**224 60 Lund (SE)**

(56) References cited:
WO-A1-00/59377        US-A- 5 002 065
US-A1- 2009 082 694    US-A1- 2018 233 230

• **GRIFFITH KATHLEEN A ET AL: "Evaluation of**
**chemotherapy-induced peripheral neuropathy**
**using current perception threshold and clinical**
**evaluations", SUPPORTIVE CARE IN CANCER,**
**SPRINGER BERLIN HEIDELBERG, BERLIN/**
**HEIDELBERG, vol. 22, no. 5, 21 December 2013**
**(2013-12-21), pages 1161 - 1169, XP035365031,**
**ISSN: 0941-4355, [retrieved on 20131221], DOI:**
**10.1007/S00520-013-2068-0**

**Description**

Technical Field

The present disclosure relates to monitoring of individuals on chemotherapy and, in particular, techniques for predicting chemotherapy-induced peripheral neuropathy (CIPN).

Background Art

Chemotherapy (CTX) is a type of cancer treatment that uses one or more anticancer drugs (chemotherapeutic agents) as part of a standardized chemotherapy regimen. Chemotherapy may be given with a curative intent or may aim to prolong life or to reduce symptoms (palliative chemotherapy). It may also be employed in treatment of other diseases than cancer, for example autoimmune and inflammatory disorders such as rheumatoid arthritis, psoriatic arthritis, psoriasis, polymyositis, Crohn disease, vasculitis, lupus, multiple sclerosis, AL amyloidosis, etc. Chemotherapy is also used in conditioning regimens prior to bone marrow transplant.

Chemotherapy-induced peripheral neuropathy (CIPN) is a common and severe side-effect of chemotherapy. CIPN is a progressive, enduring, and often irreversible condition featuring pain, numbness, tingling and sensitivity to cold in the hands and feet that afflicts a significant proportion of patients undergoing chemotherapy. CIPN may cause long-term handicap and hamper daily life activities. When hands are affected, patients may have difficulty handling small objects, dressing/undressing, taking care of personal hygiene, or preparing food. Reduced sensitivity in the feet may affect balance and the patient's ability to perceive blisters on the feet, which in turn may increase the risk for developing foot ulcers.

A number of chemotherapeutic agents are known to be associated with an elevated risk of neuropathy, including platinum-based antineoplastic agents (e.g., oxaliplatin, cisplatin), vinca alkaloids, epothilones (e.g., ixabepilone), taxanes, proteasome inhibitors (e.g., bortezomib) and immunomodulatory drugs (e.g., thalidomide). The prevalence is generally high. For example, oxaliplatin has been shown to cause CIPN in 95% of patients, with 20% exhibiting long-term (persistent) impaired tactile perception after completed chemotherapy. The condition is regarded chronic if it persists one year after end of the treatment.

Although the prime objective of chemotherapy is to save the patient's life by reverting or eliminating cancer cells, the negative impacts of chemotherapy are recognized by the medical profession. In most clinics, the patients are therefore monitored for symptoms of peripheral neuropathy during chemotherapy. Based on the monitoring, the clinician may modify the treatment regimen, for example by dose reduction, or switch to another chemotherapeutic agent, or even discontinue chemotherapy.

In current practice, symptoms of peripheral neuropathy are often quantified with patient questionnaires focused on perceived neuropathy symptoms and experienced reduction of daily life capabilities. Many different scales are available for quantifying the symptoms, including NTI-CTCAE, NCI-CTC, DEB-NTC, the Oxaliplatin Scale, EORTC QLQ-CIPN20, etc.

Self-reporting through a questionnaire is a fast and cheap. However, self-reporting is fundamentally subjective and thereby prone to misunderstanding and psychological bias of over- or underestimation, for example depending on the patient's mood or willingness to admit to having a problem. Moreover, results are found to differ between scales, and there is no consensus among medical professionals on the most appropriate scale to use. Further, symptoms may come and go during treatment, causing symptoms experienced during treatment to correlate weakly with long-term symptoms. It is therefore difficult for the clinician to accurately identify the patients that are likely to develop long-term neuropathy. It is not unlikely that chemotherapy is unnecessarily adjusted for some patients, potentially resulting in inadequate cancer treatment, while a need to adjust chemotherapy is overlooked for other patients, potentially resulting in permanent nerve damage.

Besides using questionnaires, clinical tests may be performed. An evaluation of different clinical tests is presented in the article "Quantitative Sensory Testing at Baseline and During Cycle 1 Oxaliplatin Infusion Detects Subclinical Peripheral Neuropathy and Predicts Clinically Overt Chronic Neuropathy in Gastrointestinal Malignancies", by Reddy et al, published in Clinical Colorectal Cancer, Vol. 15, No. 1, 37-46 (2016). Here, Quantitative Sensory Testing (QST) is applied for the purpose of identifying early biomarkers of the development of CIPN in patients treated by oxaliplatin. QST is a panel of diagnostic tests used to assess somatosensory function and involves a broad range of different sensations. Cutaneous sensation thresholds were measured by use of Von Frey monofilaments. Pain sensation was measured by applying a sharp tip onto the skin. Vibration perception (aka vibrotactile perception) was measured with a 64-Hz graduated Rydell-Seiffer tuning fork. Thermal tests assessed sensitivity and tolerance to extreme heat and cold. Manual dexterity and fine motor skills were also quantified. The study of the above-identified article identified cold and heat detection thresholds, as well as cutaneous detection threshold, as promising early biomarkers for the development of CIPN. No clear relationship with CIPN was found for pain sensation or vibration perception.

It should be noted that the task of predicting the risk for CIPN in a patient is distinct from the task of detecting presence of peripheral neuropathy in a patient. Detection of peripheral neuropathy involves determining an existing state of the patient, for example based on self-reporting and/or medical examination. There is no basis for an assumption that any and all input data to a physician's diagnosis of existing peripheral neuropathy would also be useful in identifying patients at risk for developing peripheral neuropathy at a future time point as a result of chemotherapy. For example, the prior art as referenced above clearly points away from using biomarkers based on pain sensation and vibration perception for such prediction.

There is a continued need for techniques capable of identifying patients that are likely to develop CIPN, based on one or more biomarkers given by measurements performed on the patients before or during chemotherapy. It is preferred for these techniques to be time efficient to avoid that the patients need to undergo a lengthy examination and multiple tests.

Summary

It is an objective to at least partly overcome one or more limitations of the prior art.

A further objective is to provide a technique for identifying patients at risk for developing CIPN.

Another objective is to provide such a technique which is based on measurement data acquired on the patient before or during chemotherapy.

A still further objective is to provide such a technique which is non-invasive and time efficient.

One or more of these objectives, as well as further objectives that may appear from the description below, are at least partly achieved by a device and method in accordance with the independent claims, embodiments thereof being defined by the dependent claims.

The invention is defined by the appended claims. A first aspect of the present disclosure is a prediction device. The prediction device comprises circuitry configured to predict a risk of chemotherapy-induced peripheral neuropathy, CIPN, in a test subject. The circuitry is configured to receive input data comprising perception data that designates measured perception of vibrations at one or more predetermined locations on one or more limbs of the test subject, wherein the perception data represents, for vibrations at each of one or more predefined frequencies, a vibration energy that causes the test subject to switch between perception and non-perception of the vibrations, wherein at least one of the one or more predefined frequencies is below 64 Hz. The circuitry is further configured to operate at least one prediction model on the perception data to determine at least one risk variable, which is indicative of a risk that the test subject will develop peripheral neuropathy as a result of chemotherapy, and generate prediction data based on the at least one risk variable.

The first aspect is based on the surprising finding that the measured perception by a test subject of vibrations at one or more vibration frequencies below 64 Hz contains information about the likelihood that the test subject will develop peripheral neuropathy, at a future time point, as a result of chemotherapy. This means that an appropriately configured prediction model may be operated on one or more biomarkers representing such measured perception, also known as vibrotactile perception, to predict the risk of chemotherapy-induced peripheral neuropathy, i.e. CIPN. The prediction device of the first aspect provides a long sought-after technique for identifying patients at risk for developing CIPN. Further, the diagnostic ability of the prediction device need not rely of subjective self-reporting by the patient, but may be based on the vibrotactile perception of the patient as quantified by measurement before or during chemotherapy. Such vibrotactile may be measured by existing equipment in a non-invasive and time efficient manner.

A second aspect is a computer-implemented prediction method. The prediction method comprises receiving input data comprising perception data that designates measured perception of vibrations at one or more predetermined locations on one or more limbs of a test subject, wherein the perception data represents, for vibrations at each of one or more predefined frequencies, a vibration energy that causes the test subject to switch between perception and non-perception of the vibrations, wherein at least one of the one or more predefined frequencies is below 64 Hz. The prediction method further comprises operating a prediction model on the perception data to determine at least one risk variable, which is indicative of a risk that the test subject will develop peripheral neuropathy as a result of chemotherapy, and generating prediction data based on the at least one risk variable.

A third aspect is a computer-readable medium comprising computer instructions which, when executed by a processor, cause the processor to perform the prediction method of the second aspect or any of its embodiments.

A fourth aspect is a prediction method. The prediction method comprises determining perception data that designates measured perception of vibrations at one or more predetermined locations on one or more limbs of a test subject, wherein the perception data represents, for vibrations at each of one or more predefined frequencies, a vibration energy that causes the test subject to switch between perception and non-perception of the vibrations, wherein at least one of the one or more predefined frequencies is below 64 Hz. The prediction method further comprises operating the prediction device of the first aspect on the perception data to generate prediction data indicative of the risk that the test subject will develop peripheral neuropathy as a result of chemotherapy.

Still other objectives, aspects, and technical effects, as well as features and embodiments may appear from the following detailed description, from the attached claims as well as from the drawings.

Brief Description of the Drawings

Embodiments will now be described in more detail with reference to the accompanying drawings.

FIG. 1A is a schematic side view, partly in section, of an example apparatus for measuring vibrotactile perception, FIG. 1B illustrates a system for measurement of vibrotactile perception thresholds (VPTs), FIG. 1C shows an example of a vibrogram provided by the system in FIG. 1A, and FIG. 1D is a flow chart of an example method of determining VPTs.

FIG. 2A is a block diagram of an example device for CIPN prediction, FIG. 2B is a flow chart of an example method of performing CIPN prediction by use of low-frequency vibration perception data (LF-VPD), and FIG. 2C exemplifies determination and use of LF-VPD in relation to sessions in chemotherapy.

FIGS 3A-3B are functional block diagrams of prediction modules with different numbers of prediction models.

FIGS 4A-4C are functional block diagrams of prediction modules with different classification functionality.

FIG. 5 is a flow chart of a procedure for evaluating adequacy of input data for CIPN prediction.

FIG. 6 is a flow chart of an example clinical use of risk classes provided by the CIPN prediction.

FIG. 7 illustrates example use of a combination of prediction modules for CIPN prediction based on time-separated input data.

FIGS 8A-8C are ROC plots determined for example prediction modules.

FIG. 9 is a block diagram of a machine that may implement any method, procedure, function, or step described herein.

Detailed Description of Example Embodiments

Embodiments will now be described more fully hereinafter with reference to the accompanying drawings, in which some, but not all, embodiments are shown. Indeed, the subject of the present disclosure may be embodied in many different forms and should not be construed as limited to the embodiments set forth herein; rather, these embodiments are provided so that this disclosure may satisfy applicable legal requirements. Like numbers refer to like elements throughout.

Also, it will be understood that, where possible, any of the advantages, features, functions, devices, and/or operational aspects of any of the embodiments and/or contemplated herein may be included in any of the other embodiments described and/or contemplated herein, and/or vice versa. In addition, where possible, any terms expressed in the singular form herein are meant to also include the plural form and/or vice versa, unless explicitly stated otherwise. As used herein, "at least one" shall mean "one or more" and these phrases are intended to be interchangeable. Accordingly, the terms "a" and/or "an" shall mean "at least one" or "one or more", even though the phrase "one or more" or "at least one" is also used herein. As used herein, except where the context requires otherwise owing to express language or necessary implication, the word "comprise" or variations such as "comprises" or "comprising" is used in an inclusive sense, that is, to specify the presence of the stated features but not to preclude the presence or addition of further features in various embodiments of the invention, which is defined by the appended claims. Similarly, the expressions "as a function of" and "based on" in combination with a specified set of parameters or the like are inclusive and do not to preclude the presence or addition of further parameters.

As used herein, the terms "multiple", "plural" and "plurality" are intended to imply provision of two or more elements. The term "and/or" includes any and all combinations of one or more of the associated listed elements.

As used herein, "prediction" refers to a process of determining, at a current time point, the likelihood that a human subject is in a specific state at a future time point.

As used herein, "chemotherapy", CTX, refers to any therapy that involves administration of a chemotherapeutic agent to a human subject for any therapeutic purpose. CTX is not limited to cancer treatment but may be applied for treatment of other diseases such as autoimmune and inflammatory disorders, and in preparation for a bone marrow transplant. The chemotherapeutic agent may be any type of neurotoxic substance. For example, the chemotherapeutic agent may be a cytotoxic or antineoplastic drug that inhibits mitosis or induces DNA damage.

As used herein, "chemotherapy-induced peripheral neuropathy", CIPN, refers to the occurrence of peripheral neuropathy in a human subject as a result of chemotherapy. In this context, "peripheral neuropathy" refers to conditions that result from damage to the nerves outside of the brain and spinal cord (peripheral nerves). Symptoms of peripheral neuropathy may include pain, numbness, tingling, sensitivity to cold, and loss of sensitivity. Peripheral neuropathy may affect any peripheral portion of the body but is usually first noticed in hands and/or feet.

It is known in the art to measure the vibrotactile perception of a human subject. The measured vibrotactile perception may be used by a physician as input to a medical analysis for detecting an ongoing sensory impairment in the human subject, for example as a result of vibration exposure or diabetes. In one simple example, the measurement is done by applying a tuning fork to the skin causing it to vibrate at its resonance frequency, typically around 100 Hz, and asking the subject to report if the vibration is perceived. It is also known to measure the vibrotactile perception by use of a specialized apparatus known as vibrometer, biothesiometer, pallesthesiometer or neurothesiometer. This apparatus is configured to measure the vibrotactile perception threshold (VPT) at one or more vibration frequencies.

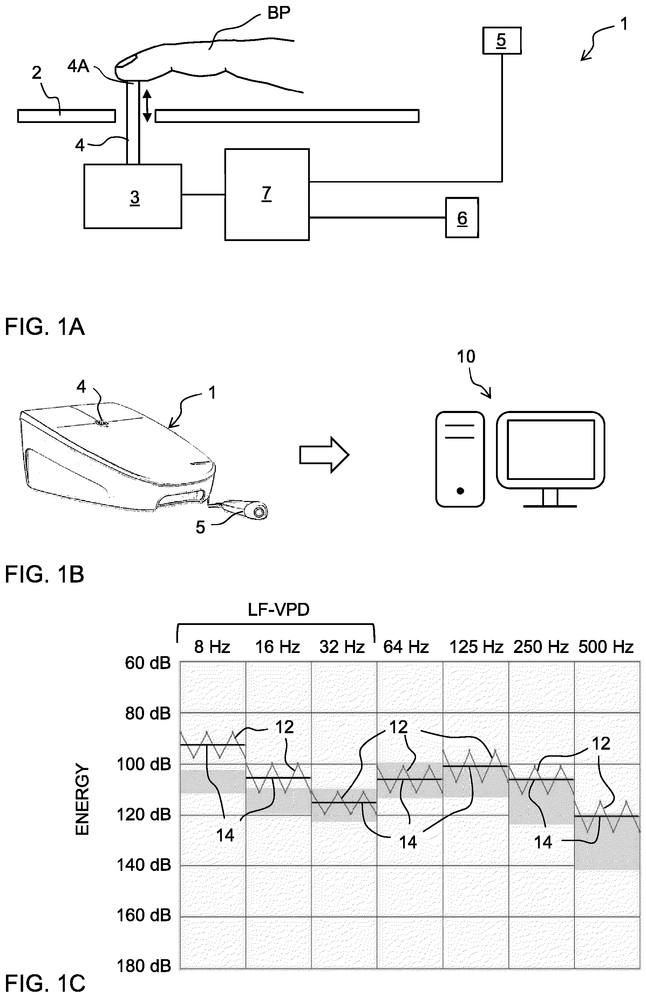

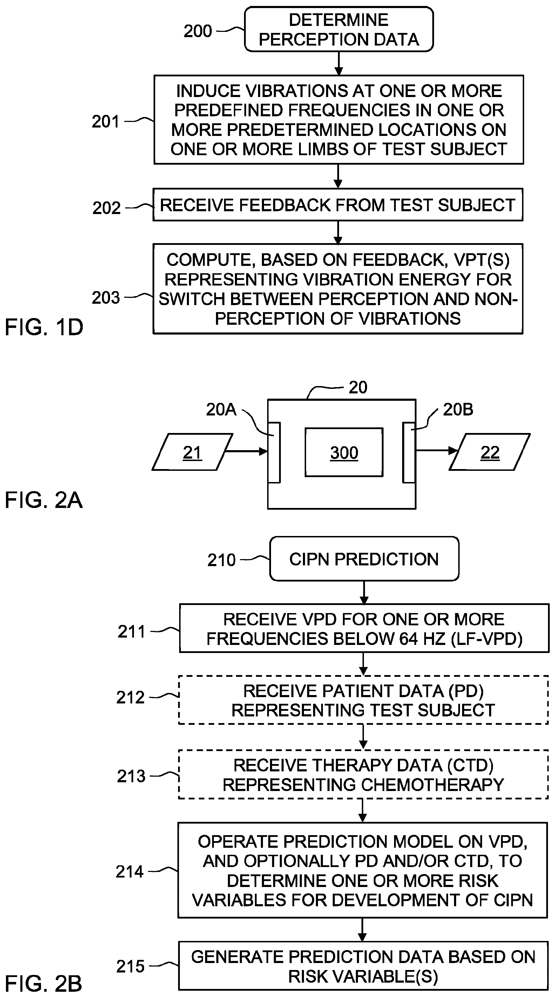

FIG. 1 is a schematic view of such an example apparatus 1, denoted "vibrometer" in the following. The vibrometer 1 is configured to generate measurement data representing the vibrotactile perception at one or more frequencies for a body part BP of a human subject to be tested, denoted "patient" or "test subject" in the following. The measurement data may be generated for any body part, including a hand or a foot. In the illustrated example, the body part BP is a hand, and the vibrotactile perception is measured for a finger on the hand. The vibrometer shown in FIG. 1A may, for example, be configured as described in WO2006/046901.

The vibrometer 1 comprises a housing 2, only shown in part, which encloses an electro-mechanical measurement system and defines an opening. The housing 2 may also serve as a mechanical support for the body part BP to improve the comfort of the patient and to ensure that the measurement data is generated in a consistent and repeatable way. The measurement system includes a measurement device 3 with a vibration probe 4 which is arranged to project through the opening to expose the distal probe end 4A. The measurement device 3 includes a vibrator (not shown), which is an electro-dynamic device that vibrates when supplied with a current or voltage, and may also include a force sensor (not shown). The probe 4 is coupled to the vibrator which is thereby operable to impart a longitudinal vibration to the probe 4, as indicated by a double-ended arrow. The force sensor may be directly or indirectly coupled to the probe 4 to sense the longitudinal application force onto the probe end 4A. The vibrometer 1 further includes a feedback device 5 which allows the patient to signal it senses the vibration of the probe 4. The feedback device 5 may include any combination of a button, keyboard, keypad, touch screen, microphone, gesture recognition system, etc. Other types of feedback devices are conceivable. An instruction device 6 enables the vibrometer 1 to output indications or instructions to the patient or an operator of the system 1. The instruction device 6 may include any combination of an indicator lamp, display, speaker, etc. A control unit 7 operates the vibrometer 1 and is connected, by wire or wirelessly, to provide a control signal to the measurement device 3 and an instruction signal to the instruction device 6, and to receive a feedback signal from the feedback device 5.

The control unit 7 is configured to perform a test procedure to generate the measurement data. During the test procedure, the control unit 7 operates the measurement device 3 to impart vibrations of different energies to the probe 4 at a predefined frequency. The patient, which has engaged a finger, e.g. the fingertip as shown, with the probe end 4A, then indicates via the feedback device 5 when the vibration of the probe 4 is sensed. This measurement may be repeated for one or more additional frequencies, resulting in measurement data for multiple frequencies. During the test procedure, the control unit 7 may monitor the force signal to ensure that the application force of the fingertip on the probe end 4A is adequate.

FIG. 1B shows an implementation of the vibrometer 1, in which the feedback device 5 is a handle with a push button. In the illustrated example, the measurement data is transferred, for example by wire or wirelessly, from the vibrometer 1 to an analysis device 10, which may be any type of computation device.

FIG. 1C is a so-called vibrogram, which has been generated for a test procedure on a patient. In FIG. 1C, the vertical axis represents vibration energy. The horizontal axis is separated into different predefined frequencies, in this example 8 Hz, 16Hz, 32 Hz, 64 Hz, 126 Hz, 250 Hz, and 500 Hz. For each of the predefined frequencies, FIG. 1C shows a curve 12 representing vibration energy imparted to the probe 4 as a function of time. In the illustrated example, the curve 12 forms a zigzag pattern, which implies that the vibration energy has been alternately increased and decreased. Concurrently, the feedback from the patient is registered. The feedback may indicate that perception ends during a gradual decrease in vibration energy and/or that perception starts during a gradual increase in vibration energy. Based on this feedback, a vibrotactile perception threshold (VPT) may be computed. In FIG. 1C, the VPT is represented by a horizontal line 14 and thus corresponds to a vibration energy. The computation of VPTs is well-known in the art and will not be described in further detail. For example, reference is made to the ISO standard for vibrotactile perception measurements, given by ISO 13091-1 and 13091-2.

In an alternative, the VPT may be represented as a so-called z-score, also known as a standard score. The z-score represents the number of standard deviations by which a data point is above (positive) or below (negative) a mean value of data points. For example, z-scores may be computed to normalize the VPTs for parameters such as age, gender, etc., by the mean value representing an average of VPTs measured for a selected population of patients at a predefined frequency. The selected population may, e.g., be selected to have the same age, gender and biometric data (e.g., height, finger temperature) as the patient. The patients in the selected population may be chosen on the basis of having normal (undamaged) sensitivity to vibrations.

FIG. 1D is a flow chart of an example method 200 of determining one or more VPTs for a patient. The method 200 may be performed by the vibrometer 1 in combination with the analysis device 10. Alternatively, the method 200 may be performed by the vibrometer 1 itself.

Step 201 induces vibrations at one or more predefined frequencies in one or more predetermined locations on one or more limbs of the patient, for example by the probe 4 in FIG. 1A, while varying the vibration energy. Step 202 receives, during step 201, feedback from the patient whether the vibrations are perceived or not. Steps 201-202 may be performed as a method of limits or a method of levels. In the method of limits, the stimulus (vibration energy) is gradually increased or decreased (cf. 12 in FIG. 1C) and the test subject provides feedback when the stimulus is first perceived during stimulus increase or no longer perceived during stimulus decrease. In the method of levels, the stimulus is instead applied repeatedly below and above a threshold value, and the test subject is asked to provide feedback to indicate if the respective stimulus is perceived or not. As also understood from the foregoing, steps 201-202 are performed separately for each predefined frequency and each predetermined location. Step 203 computes, based on the feedback, at least one VPT to represent, for each frequency and location, the vibration energy when the patient experiences the switch from perception to non-perception, or from non-perception to perception, or both. It should be noted that more than one VPT may be computed for a frequency and location, for example if step 201 comprises generates more than one instance of measurement data for the frequency and location. Step 203 may also comprise a computation of one or more z-scores based in the VPT(s).

In a variant, the measurement according to steps 201-202 may be performed simultaneously for more than one location and/or body part. For example, the vibrometer may be provided with a plurality of vibrating probes, or a vibrating plate, which is engaged with two or more locations on a body part (for example, plural fingers on one hand) and/or one or more locations on two or more body parts (for example, the same finger on different hands). It is also conceivable that the vibrating probe/plate is controlled to vibrate at two or more frequencies at the same time.

FIG. 2A is a schematic view of an example prediction device 20 which is configured to predict, based on input data 21, the risk that a patient will experience CIPN at a future time point. The prediction device 20 may be configured to operate in accordance with any of the methods, processes, routines or steps presented herein. In the illustrated example, the prediction device 20 comprises an input interface 20A for receiving the input data 21, a prediction module 300, and an output interface 20B for providing prediction data 22, which indicative of a predicted risk for CIPN. The prediction data 22 may be output from the prediction device 20 for storage in a memory, for display on a display unit, for further processing, etc. The prediction device 20 may be any type of computer device, including but not limited to a desktop computer, a laptop, a tablet, a smartphone, etc. The input data 21 includes vibration perception data (VPD) which represents one or more measurements of vibrotactile perception on the patient. Depending on implementation, the VPD may include the above-mentioned measurement data, or parameter data derived from the measurement data, such as one or more VPTs, one or more z-scores, etc. The prediction device 20 may be part of the analysis device 10 (FIG. 1B) or the vibrometer 1 (FIG.

1B), or be a separate device. It is realized that the interfaces 20A, 20B may be internal or external interfaces, and that the input data 21 may be provided by electronic transfer or manual entry, or any combination thereof.

FIG. 2B is a flow chart of an example method 210, which may be performed by the prediction device 20 to generate the prediction data 22. The method 210 is the result of significant experimentation, which led to the surprising finding that measurements of vibrotactile perception may be indicative of a future risk for CIPN, if the measurements are made at vibration frequencies below about 64 Hz. In the following, VPD obtained for one or more frequencies below 64 Hz is denoted "low-frequency VPD", or LF-VPD. As indicated in the vibrogram of FIG. 1C, the measurements at 8 Hz, 16 Hz and 32 Hz result in LF-VPD. It is presently contemplated to perform CIPN prediction based on LF-VPD for frequencies at or below 60 Hz, 50 Hz, 40 Hz, 35 Hz, 30 Hz, 25 Hz, 20 Hz, 15 Hz, or 10 Hz. For practical reasons, the vibration frequencies may be further restricted to be above 0.1 Hz, or 0.5 Hz.

The method 210 may be performed before and/or during chemotherapy. Step 211 receives VPD for one or more frequencies below 64 Hz, i.e. LP-VPD. In addition to LP-VPD, step 211 may receive VPD for frequencies at or above 64 Hz. As indicated by dashed lines, the method 210 may further comprise optional steps 212-213. Step 212 receives patient data (PD) that represents the patient. Step 213 receives therapy data (CTD) that represent the prescribed chemotherapy (on-going, upcoming, historic) for the patient. The patient data and the therapy data may be in the form of a set of parameter values. Examples of PD and CTD are given further below. In the example of FIG. 2A, the VPD is included in the input data 21, optionally together with PD and/or CTD.

The following description assumes that the VPD is represented in the input data 21 in the form of one or more "perception values", which are representative of the measured vibrotactile perception of the patient. For example, the perception values may be VPTs or z-scores. If the VPD instead is represented in the form the above-mentioned measurement data, the method may comprise a step similar to step 203 in FIG. 1D, which computes one or more perception values from the measurement data.

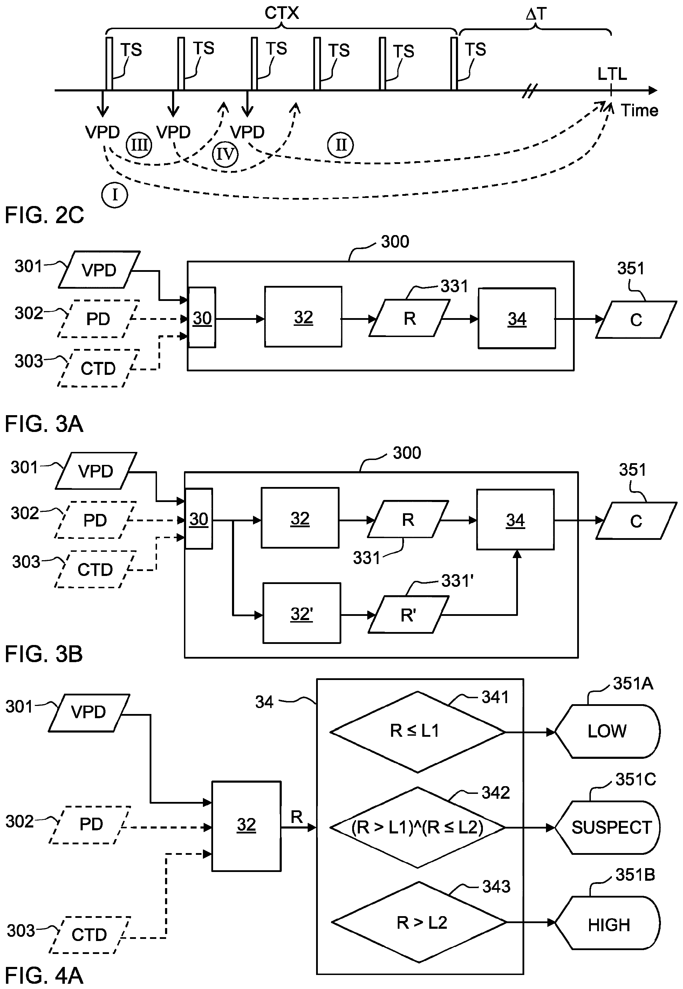

The method 210 further comprises step 214, which operates a prediction model on the VPD to determine at least one risk variable, which is indicative of the risk that the patient will develop peripheral neuropathy as a result of the chemotherapy. If available, step 214 may operate the prediction model also on PD and/or CTD to determine the risk variable(s). Step 215 generates prediction data (cf. 22 in FIG. 2A) based on the risk variable(s). In some embodiments, the prediction data is generated to include the risk variable(s). In some embodiments, the prediction data is generated by processing the risk variable(s), as will be further exemplified below with reference to FIGS 3-4.

The utility of the prediction method 210 will be further described with reference to FIG. 2C, which schematically shows an example of chemotherapy (CTX) prescribed for a patient. CTX is commonly given at regular intervals called cycles. A cycle may be a dose of one or more chemotherapeutic agents on one or more days, followed by several days or weeks without treatment ("rest period"). The rest period gives normal cells time to recover from side effects, which include transient peripheral neuropathy. Sometimes, doses may be given a certain number of days in a row, or every other day for several days. Some chemotherapeutic agents work best when given continuously over a set number of days. In the present disclosure, a cycle with administration of one or more doses is denoted a "therapy session" or "session". Any two sessions are separated by a rest period. In the example of FIG. 2C, CTX consists of a time sequence of six therapy sessions, TS. CTX is typically given by a so-called chemotherapy regimen ("CTX regimen"), which is defined by the clinician in charge and specifies the chemotherapeutic agent(s), their dosage, the frequency and duration of treatment sessions, the method of administrating the chemotherapeutic agent (injection, tablets, etc.), and other considerations.

FIG. 2C also indicates, by dashed arrows I-IV, different ways of using the prediction method 210 in relation to VPD measured before and during CTX. In alternative I, the method 210 is operated on VPD measured before the initial TS, i.e. before start of CTX, to predict the risk for CIPN at a long-term time limit, LTL, which may be set a given time period, $\Delta T$, after completed CTX, for example 6 or 12 months. Such long-term CIPN should be avoided since it is likely to have a lasting impact on the patient. Alternative I enables the clinician to tailor the CTX regimen to avoid or reduce long-term CIPN. In alternative II, the method 210 is operated on VPD measured during CTX, in this example before the third TS, to predict the risk for long-term CIPN. Alternative II enables the clinician to follow-up the risk for long-term CIPN during CTX and adjust the CTX regimen accordingly. In alternative III, the method 210 is operated on VPD measured before the initial TS to predict the risk for CIPN during CTX ("short-term CIPN"). Alternative III enables the clinician to tailor the CTX regimen to reduce discomfort of the patient during CTX and also to reduce the risk for long-time CIPN, which is likely to correlate to some degree with short-term CIPN. In alternative IV, the method 210 is operated on VPD measured during CTX, in this example before the second TS, to predict the risk for CIPN later on during CTX, in this example before the fourth TS. Alternative IV, like alternative II, allows the clinician to follow-up and adjust the CTX regimen. Although not shown in FIG. 2C, it is also conceivable to operate the method 210 on a combination of VPDs measured at different time points. Generally, such a combination of VPDs may be measured prior to each of at least two sessions and/or at two or more time points between sessions. In one example, the combination includes VPD measured before the first TS and VPD measured before the second TS. In another example, the combination includes VPD measured at different time points during a single rest period. In a further example, the combination includes VPD measured at time points during different rest periods.

It has been found that the accuracy of the prediction data (22 in FIG. 2A) may be increased by including therapy data, CTD, in the input data (21 in FIG. 2A) and by operating the prediction model on a combination of VPD and CTD (step 214 in FIG. 2B). The CTD may represent the CTX regimen and may, for example, include one or more parameters representative of the chemotherapeutic agent(s), the dose, or the schedule. In an example, the dose may be given as the accumulated dose that is to be administered during the CTX. In a further example, the schedule may be given as a number of sessions, a duration of the respective session, a duration of the rest period, or any combination thereof. The CTD may also include the CTX treatment history of the patient, for example the CTX regimen of a previous CTX, time since last CTX, time since last treatment session in an ongoing CTX, etc. The use of both VPD and CTD has the additional advantage of allowing the clinician to evaluate the risk for CIPN in relation to different CTX regimens for the patient, by modifying the CTD.

It has also been found that the accuracy of the prediction data (22 in FIG. 2A) may be increased by including patient data, PD, in the input data (21 in FIG. 2A) and by operating the prediction model on a combination of VPD and PD (step 214 in FIG. 2B). Examples of parameters that may be included in PD are age, gender, physical characteristics, current temperature, health status, medication status, and medical history. The physical characteristics may be one or more of height, weight, BMI, obesity, etc. The current temperature is the temperature of the patient when the VPD is measured, for example the temperature of the body part for which VPD is measured. The health status is the status of the patient at the time point when the prediction is made. The health status may, for example, indicate if the patient has an existing neuropathy and/or any comorbidities, such as diabetes, Parkinson's disease, multiple sclerosis (MS), etc. The medication status may indicate any drugs that are prescribed for the patient, other than the chemotherapeutic agent(s). The medication history may also indicate the dose of such drug(s), etc. The medical history (anamnesis) may indicate any relevant illness, disease or disorder that the patient has previously suffered from, including but not limited to neuropathy. The medical history may also indicate the time since the last episode of the illness, etc. It is also conceivable that PD includes subjective data from questionnaires, for example a categorization in accordance with any of the existing scales mentioned in the Background section. Still further, PD may include data from other types of measurements on the patient, such as cold and heat detection thresholds, cutaneous detection threshold, pain sensation, etc.

It is also to be understood that the prediction may be further improved by operating the prediction model on a combination of VPD, CTD, and PD.

Improvement of prediction may also be achieved by operating the prediction model on VPD measured for more than one vibration frequency below 64 Hz. VPD at different frequencies may be measured in the same location or in different locations on the same or on different limbs. In some embodiments, the limb may be a hand or a foot, and the location may be a metatarsal head on the foot or a finger on the hand. In case a patient has a unilateral neurological disorder, the VPD may be measured on the unaffected side of the patient. It is currently believed that the precision of the prediction is improved when the input data comprises VPD measured on at least one foot of the test subject, compared to VPD measured at other sites on the human body.

Improvement of prediction may also be achieved by operating the prediction model on VPD measured in more than one location and/or on one or more limbs.

Thus, generally, improvements may be achieved by operating the prediction model on a plurality of perception values in the VPD, where the perception values differ by at least one of vibration frequency, location, or limb.

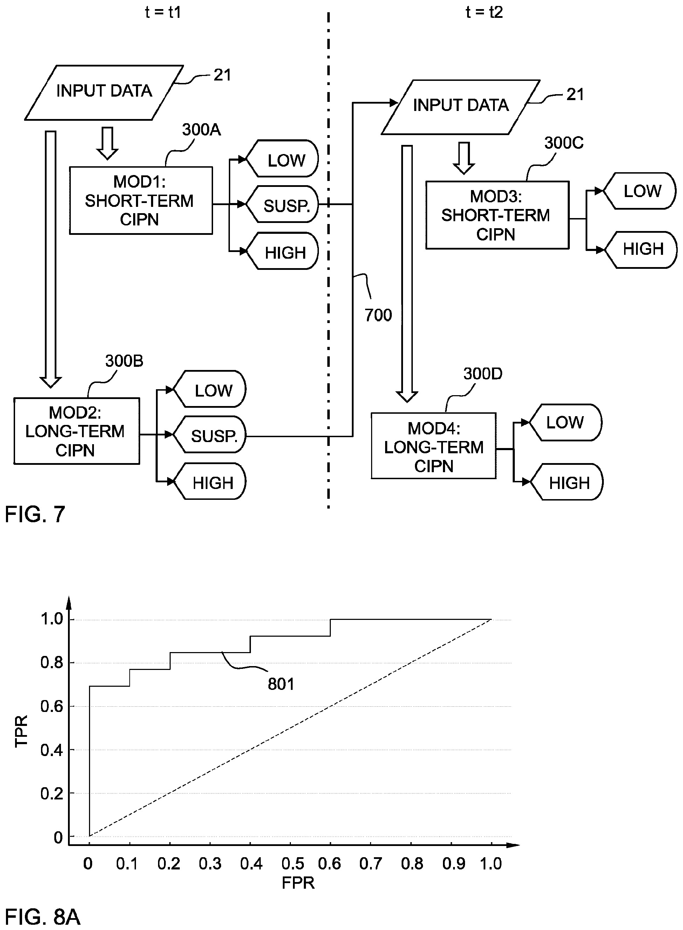

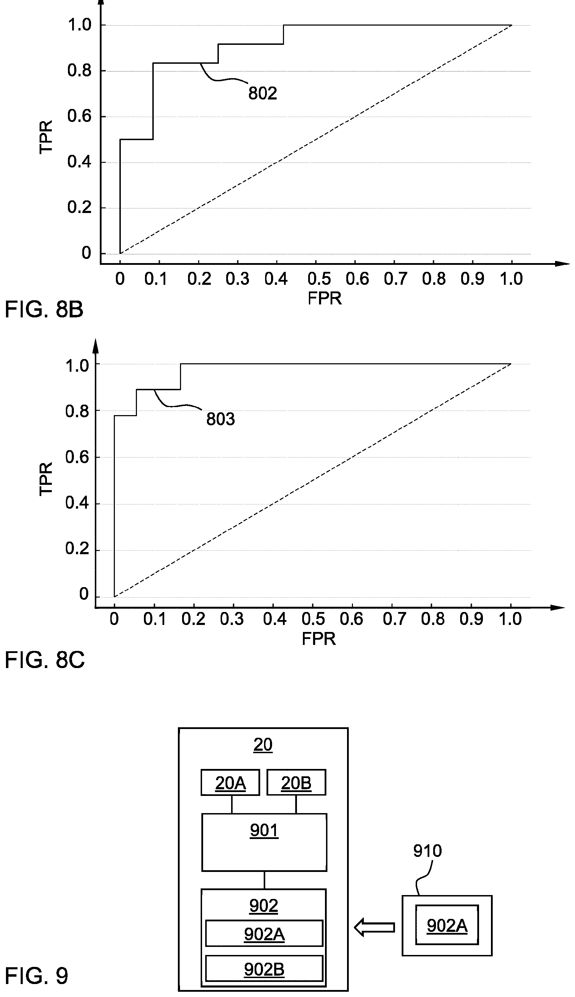

The effectiveness of the prediction method 210 is further illustrated in FIGS 8A-8C, which are so-called ROC plots. The ROC plots have been generated by operating the prediction method 210 on input data from a clinical study in which patients on CTX were evaluated for signs of CIPN, either based on self-reporting (NTI-CTCAE scale) or based on measured z-scores. The patients were separated into two groups on different combinations of drugs, both including oxaliplatin. In FIG. 8A, curve 801 is an ROC curve for prediction of long-term CIPN (6 months after completed CTX, given by self-reporting) based on VPD (z-scores for 4 Hz, 8 Hz and 16 Hz measured on the first metatarsal head on the right foot), CTD (combination of drugs, number of sessions, and accumulated dose), and PD (age). In FIG. 8B, curve 802 is an ROC curve for prediction of long-term CIPN (6 months after completed CTX, given by measured z-scores) based on VPD (z-scores for 4 Hz, 8 Hz, 16 Hz and 32 Hz measured on the first metatarsal head on the right foot), CTD (combination of drugs, and accumulated dose). In FIG. 8C, curve 803 is an ROC curve for prediction of short-term CIPN (any time during CTX, given by self-reporting) based on VPD (z-scores for 4 Hz, 8 Hz and 16 Hz measured on the first metatarsal head on the right foot), CTD (combination of drugs), and PD (gender).

The examples in FIGS 8A-8C indicate that the prediction method 201 is capable of predicting long-term and short-term CIPN with good accuracy. The examples in FIGS 8A-8C are only included to demonstrate the effectiveness of the prediction method 201, which in no way is limited to the input data used in these examples. The input data that is to be used for a specific CIPN prediction in relation to a specific CTX regimen is a tradeoff between time required to collect the input data and the resulting accuracy of the CIPN prediction. The choice of input data may be determined by testing based on clinical or simulated data.

The examples in FIGS 8A-8C are all given for CTX with administration of oxaliplatin. However, corresponding results are expected for other neurotoxic substances. According to scientific literature, the neurotoxicity of chemotherapeutic drugs is mainly caused by six different mechanisms: (i) activation of immune cells; (ii) damage of mitochondrial DNA transcription; (iii) altered activity of ion channels; (iv) damage of myelin sheath; (v) disruption of microtubule; and (vi) changed metabolism of astrocytes. This finding is, for example, known from the article "Mechanisms of Chemotherapy-Induced Peripheral Neuropathy", by Zajaczkowska et al., published in Int. J. Mol. Sci. 2019, 20, 1451. The mechanisms may act in parallel and may amplify one another. Mechanism (i) promotes inflammation of neurons, whereas mechanisms (iii)-(vi) mainly cause altered excitability of the peripheral neurons. Mechanism (ii) has both an inflammatory and a cell-damaging effect. Existing chemotherapeutic drugs with neurotoxic effect may be roughly divided into six different groups: (a) platinum-containing drugs, (b) taxanes, (c) immunomodulatory drugs, (d) vinca alkaloids, (e) epothilones, and (f) protease inhibitors. Each of these groups derives its neurotoxicity from one or more of the mechanisms (i)-(vi). Oxaliplatin belongs to group (a), which exhibits mechanisms (i), (ii), and (iii). It is currently believed that the prediction method 210 is applicable to any CTX that involves administration of a chemotherapeutic drug that exhibits at least one of the mechanisms of oxaliplatin and/or is known to result in CIPN. Thus, the method 210 should be applicable all other drugs in group (a), including but not limited to carboplatin or cisplatin. Group (b) exhibits mechanisms (i), (ii), (iii) and (iv) and is thereby also closely similar to group (a) in terms of mechanisms. Thus, the method 210 should be applicable all drugs in group (b). Groups (c)-(f) all fulfil the criterion of sharing at least one mechanism with group (a) and/or being known to result in CIPN. Thus, the method 210 should be applicable all drugs in groups (c)-(f) as well.

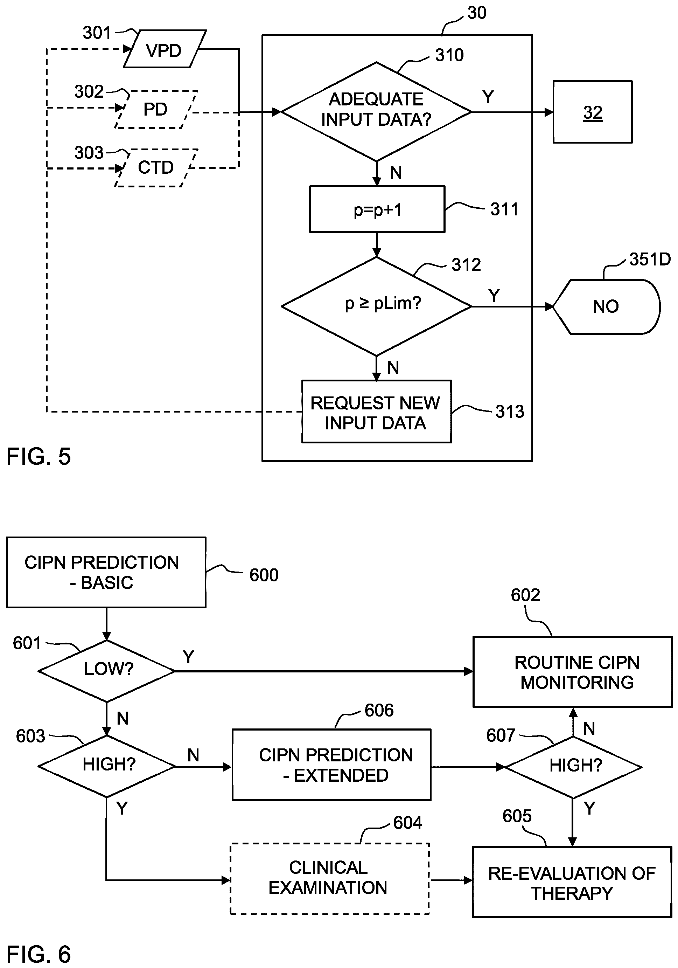

FIG. 3A is a block diagram of an example prediction module 300 for use in the prediction device 20 of FIG. 2A. In the illustrated example, the module 300 is configured to receive and process input data in the form of VPD 301, and possibly PD 302 and CTD 303, for generation of prediction data 351. The prediction data 351 comprises a classification of the risk for CIPN, designated by C. The classification C may be included as an indication of a risk class among a number of predefined risk classes, for example HIGH (high risk for CIPN) and LOW (low risk for CIPN), or as probability values for each of the predefined risk classes. The illustrated prediction module 300 comprises a pre-processing unit 30 (optional), which may be configured to condition the input data before it is processed by a prediction model 32. For example, the pre-processing unit 30 may encode the input data into a format required by the model 32 and/or generate specific parameters from the input data. The pre-processing unit 30 may also remove outliners from the input data and/or perform an adequacy check of the input data, and optionally request further input data based on the adequacy check (cf. FIG. 5, below). The prediction model 32 is configured to generate one or more risk variables 331, designated by R. The risk variable(s) 331 may be indicative of the risk that the patient will develop low-term or short-term CIPN. The respective risk variable may be given as a numeric value. The module 300 comprises an evaluation unit 34, which processes the risk variable(s) R to generate the classification C. In one example, the evaluation unit 34 compares the risk variable(s) to one or more thresholds. Examples of the processing by the evaluation unit 34 are given below with reference to FIGS 4A-4C. In some embodiments, the evaluation unit 34 may be omitted.

The prediction model 32 may define any functional relationship between risk variable(s) and variables in or given by the input data. In some embodiments, the prediction model is linear to simplify computations. A linear model may combine a set of variables by use of a plurality of predetermined weight factors into a risk variable according to:

$$r1 = \sum_{i=1}^{n} w_i \cdot v_i$$

with r1 being the risk variable, $w_i$ being the weight factors, $v_i$ being the variables, and $n$ being the number of variables. Any number of variables may be used, but $n$ is typically at least 3 and may be as large as 100. The variables may include both quantitative variables and qualitative variables. A quantitative variable originates from a measurement or counting and may, e.g., be a VPT, a z-score, a number of sessions, an accumulated dose, an age, a height, etc. A qualitative variable does not originate from a measurement and may, e.g., represent a drug or combination of drugs, a gender, a medical status, a health status, a medical history, etc. The respective quantitative variable may be given its measurement value, optionally reformatted by the pre-processing unit 30, whereas the respective qualitative variable may be given any suitable numeric value, for example in a predefined number series.

As is well-known in the art, the linear model is calibrated or "trained" based on reference data, to determine values of the weight factors $w_i$. When the weight factors have been determined, the linear model may be applied as the prediction model 32 in the module 300. The determination of the weight factors may be performed by use of any conventional mathematical method for predictive analysis of multivariate data, including but not limited to Partial Least Squares-Discriminant Analysis (PLS-DA), Sparse PLS-DA, Decision Trees (e.g., Classification and Regression Trees (CART), Random Forest Classifier, etc.), Support Vector Machines (SVM), k-Nearest Neighbors (k-NN), Naive Bayes classification, etc.

In an alternative, the prediction model 32 may be a machine learning-based model (MLM), for example involving an artificial neural network. Such a model also includes coefficients which are determined by training of the model based on reference data. The training may be performed by any conventional method.

It is to be understood that the reference data may be selected in view of the discrimination or classification to be enabled by the risk variable(s). For example, to discriminate between a set of predefined categories, the reference data should include patients that have been classified into the set of categories. The categories may but need not be mutually exclusive. Any number of categories may be used. In one example, the categories are "long-term CIPN" and "no CIPN". In another example, the categories are "short-term CIPN" and "no CIPN". In another example, the categories are different severities of CIPN, for example given by any of the existing scales mentioned in the Background section. In another example, the categories are "low risk for CIPN" and "not low risk of CIPN". In another example, the categories are "high risk for CIPN" and "not high risk of CIPN". In another example, the categories are different symptoms of CIPN, for example "tingling sensation", "pain", etc.

FIG. 3B is a block diagram of a variant of the prediction module 30 in FIG. 3A. The module 300 comprises a first prediction model 32 and a second prediction model 32'. The first model 32 is configured to generate one or more first risk variables 331, designated by R, and the second model 32' is configured to generate one or more second risk variables 331', designated by R'. The evaluation unit 34 is configured to determine the classification by a joint evaluation of R and R'. Such a joint evaluation may be done in parallel or in sequence, or both. By using two models and performing the joint evaluation, the accuracy of the prediction may be improved. This improvement is not merely an effect of providing additional risk variables, but also since the use of two models allows the models to be optimized for different types of discrimination, which may increase both sensitivity and specificity of the module 300. More than two prediction models may be used.

The first and second models 32, 32' may differ by the variables that are included in the respective model. The variables may but need not at least partly overlap between the models 32, 32'. Preferably, both models 32, 32' operate on at least one variable that represents LF-VPD. The number of variables (n) may differ between the models 32, 32'. Additionally or alternatively, the models 32, 32' may differ by the discrimination or classification that is enabled by the risk variable(s). Additionally or alternatively, the models 32, 32' may differ by the mathematical model used for calibrating or training the respective model. Additionally or alternatively, the models 32, 32' may be of different types, for example a linear model and a non-linear model, or a linear model and an MLM.

As noted above, the prediction data may be a risk classification C of the patient. The classification C may indicate one of a set of predefined risk classes. For example, the predefined risk classes may correspond to the above-mentioned categories. For example, C may be either "long-term CIPN" and "no CIPN", either "short-term CIPN" or "no CIPN", a set of severity categories of CIPN, either "low risk for CIPN" or "not low risk of CIPN", either "high risk for CIPN" or "not high risk of CIPN", a set of symptom categories, etc.

In some embodiments, the prediction module is configured to generate the prediction data to be indicative of one of at least three predefined risk classes. This will improve the usability of the prediction data for evaluating the patient.

In some embodiments, the at least three predefined risk classes comprise a first risk class associated with a low risk, a second risk class associated with a high risk, and a third risk class intermediate the first and second risk classes. The first, second and third risk classes may relate to the risk for long-term CIPN, short-term CIPN, or both. By the third risk class, the prediction data will help the clinician to discriminate between patients that need to have their CTX regimen adjusted (second risk class) and the patients that merely need to be monitored more closely (third risk class). This has the potential of saving significant medical resources.

In some embodiments, the prediction module 300 is configured to determine a risk category based on a comparison of the risk variable(s) to at least one predetermined threshold, and generate the prediction data based on the risk category. In the examples of FIGS 3A-3B, this processing is performed by the evaluation unit 34. The two-step procedure of going from risk variable to risk category, and from risk category to prediction data, makes it possible to improve the accuracy of the prediction data. This advantage will be exemplified with reference to FIGS 4A-4C.

In the example of FIG. 4A, the module 300 includes a prediction model 32 that generates a risk variable R which is indicative of the risk for the patient to develop CIPN. The evaluation unit 34 is configured to define three decision steps 341-343 in which R is compared to first and second thresholds L1, L2 ("discrimination thresholds"). If step 341 finds that $R \leq L1$, the patient is given risk category "LOW" and the prediction data is set to risk class 351A, "LOW" (first risk class). If step 342 finds that $R > L2$, the patient is given risk category "HIGH", and the prediction data is set to risk class 351B, "HIGH" (second risk class). Otherwise, if step 343 finds that $L1 < R \leq L2$, the patient is given risk category "SUSPECT", and the prediction data is set to risk class 351C, "SUSPECT" (third risk class). It is realized that one of steps 341-343 may be implicit.

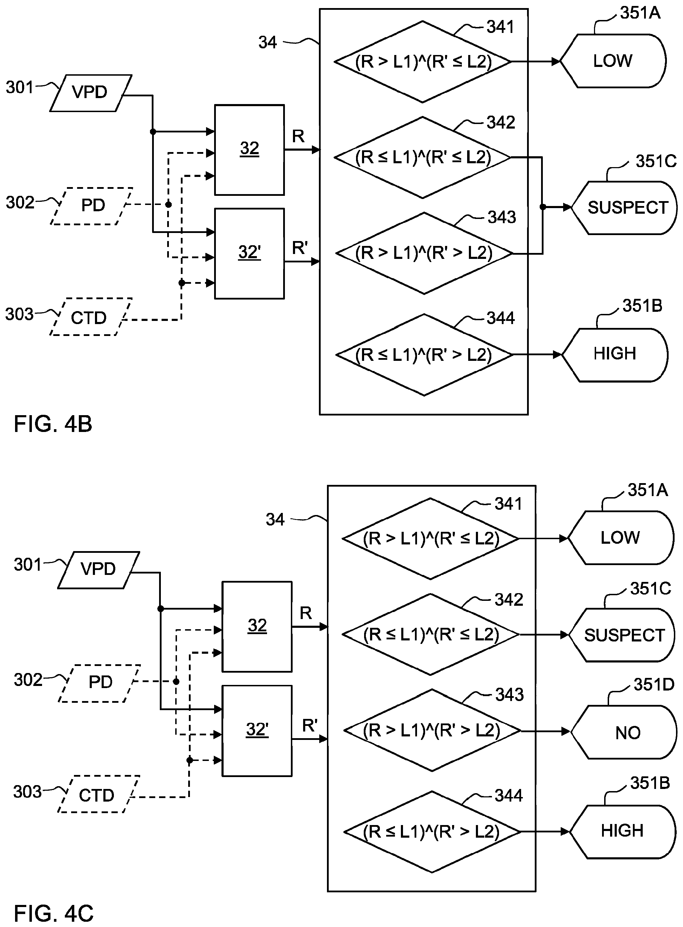

In the example of FIG. 4B, the module 300 includes two prediction models 32, 32', which each generates a risk variable R, R'. Risk variable R is generated to discriminate between risk categories "LOW" and "NOT LOW". Risk variable R' is generated to discriminate between risk categories "HIGH" and "NOT HIGH". The evaluation unit 34 is configured to define four decision steps 341-344 in which R is compared to a first threshold L1, and R' is compared to a second threshold L2. Steps 341-344 correspond to a logic combination of a risk category determined for R (by comparison to L1) and a risk category determined for R' (by comparison with L2). If $R > L1$, the risk category is "LOW". If $R \leq L1$, the risk category is "NOT LOW". If $R' > L2$, the risk category is "HIGH". If $R' \leq L2$, the risk category is "NOT HIGH". In the example of FIG. 4B, step 341 sets the prediction data to risk class 351A, "LOW", if R yields category "LOW" and R' yields category "NOT HIGH". Step 342 sets the prediction data to risk class 351C, "SUSPECT", if R yields category "NOT LOW" and R' yields category "NOT HIGH". Step 343 sets the prediction data to risk class 351C, "SUSPECT", if R yields category "LOW" and R' yields category "HIGH". Step 344 sets the prediction data to risk class 351B, "HIGH", if R yields category "NOT LOW" and R' yields category "HIGH".

It is realized that higher sensitivity and specificity of the risk classes "LOW" and HIGH" are enabled in FIG. 4B compared to FIG. 4A, by the logic combination of risk categories determined for two risk variables R, R'.

It should be noted that the example in FIG. 4B may be implemented by first determining risk categories for R and R', and then evaluating the risk categories for generation of the prediction data. In an alternative implementation, the determination and evaluation of risk categories are interleaved.

It should also be noted that the logic combination of risk categories may differ from FIG. 4B. In one example, model 32 may dominate model 32', by setting risk class "LOW" if R yields risk category "LOW" irrespective of the risk category for R'. In another example, model 32' may dominate model 32, by setting risk class "HIGH" if R' yields risk category "HIGH" irrespective of the risk category for R.

It is also to be understood that further risk classes may be defined for the prediction data. An example is given in FIG. 4C, which differs from FIG. 4B in that step 343 sets the prediction data to risk class 351D, "NO CLASS", if R yields category "LOW" and R' yields category "HIGH". The use of a fourth risk class may further reduce the need for medical resources, by reducing the number of patients that are assigned risk class 351C, "SUSPECT". The rationale for risk class 351D is that something is most likely wrong when R and R' yield contradictory risk categories ("LOW" and "HIGH"). Risk class 351D may thus be used to signal a potential error to the clinician, who may thereby be compelled to review and possibly update the input data.

The examples in FIGS 4B-4C are also appliable to a single prediction model 32 that is configured to generate two or more risk variables. For example, such a prediction model may generate a first risk variable which is indicative of the risk for CIPN, and a second risk variable which is a reliability score for the first risk variable. By analogy with the processing of R, R' in FIGS 4B-4C, the first risk variable may be compared to a first threshold to generate a risk category, and the second risk variable may be compared to a second threshold to generate a reliability category, and the prediction data may be generated by a logic combination of the risk category and the reliability category.

FIG. 5 is a flow chart of an adequacy checking procedure that may be performed by the pre-processing unit 30 (FIGS 3A-3B). The procedure is performed whenever the prediction module 300 receives input data. Step 310 analyzes the input data with respect to at least one requirement. The respective requirement may be defined for VPD, PD or CTD. In some embodiments, a requirement for VPD may be violated: 1) if data is missing, for example in terms of the number or type of variables; 2) if a deviation is detected in VPTs or z-scores; 3) if a force value, which is measured by the vibrometer and included in the input data, is outside a predefined range; 4) if a temperature of the body part, which is measured by the vibrometer and included in the input data, is outside a predefined range; 5) if the VPD has been generated at an incorrect location or on an incorrect limb (assuming that the location/limb is indicated in the input data); 6) if a reliability index, which is generated by the vibrometer and included in the input data, is below a limit. In some embodiments, a requirement for PD or CTD may be violated: 1) if data is missing, for example in terms of the number or type of variables; 2) if one or more variables are deemed faulty or suspect, e.g., by a value being outside a predefined range, or by non-realistic combination of values for variables, for example by a deviating ratio between height and weight of the test subject. If step 310 determines that the input data is adequate in view of the at least one requirement, the procedure proceeds to prediction by providing the input data to the prediction model 32. If the step 310 determines that the input data violates a requirement or a combination of requirements, the procedure proceeds to step 311, which increments a counter p. Step 312 compares the counter p to a limit *pLim.* If p does not exceed *pLim,* the method proceeds to step 313, which outputs a request for new input data. The request may be output to a display, microphone, etc., and may also indicate the reason for the request, for example in terms of the violated requirement(s). When new input data is received in response to the request, the procedure is repeated. If the procedure is repeated too many times so that $p \geq pLim,$ step 312 informs the operator accordingly, for example by setting the prediction data to risk class 351D, "NO CLASS", as shown. Thereby, prediction operation is aborted. It is understood that the counter p is reset when step 310 proceeds to prediction or when step 312 finds that $p \geq pLim.$ In a variant, the limit *pLim* may be different for different types of data. For example, a smaller limit may be set for VPD, which may need to be updated through a measurement on the test subject (cf. method 200 in FIG.1D), compared to PD or CTD.

It may be noted that step 310 may proceed to prediction even if certain variables are missing, faulty or suspect, if the prediction model 32 is configured to compensate for the variables, for example by using predicted values instead, as is known in the art. However, step 310 may consider one or more variables to be mandatory and proceed to step 311 if any such variable is missing, faulty or suspect. Further, step 310 may proceed to step 311 if the number of (non-mandatory) variables that are missing, faulty or suspect exceeds a limit.

FIG. 6 shows an example of how the CIPN prediction as described herein may be used in a clinical setting. The illustrated example presumes that the prediction data designates one of the risk classes "LOW", "HIGH" and "SUSPECT" (cf. 351A-351C in FIGS 4A-4B). In step 600, input data of a basic or default scope is acquired for a patient, and the prediction method 210 is operated on the default input data to generate prediction data. For example, VPD may be acquired by performing measurements in accordance with a default protocol. In addition to this VPD, the default input data may include a default set of variables of PD and/or CTD. If the prediction data generated by step 600 designates risk class "LOW", the clinician may decide (step 601) to put the patient on routine monitoring for CIPN (step 602). Such routine monitoring may involve, in connection with each treatment session, taking and analyzing blood samples and quantifying any peripheral nerve symptoms by use of patient questionnaires. If the prediction data instead designates risk class "HIGH", the clinician may decide (step 603) that the patient should undergo a clinical examination (step 604) to evaluate if the patient is likely to have a high risk of developing CIPN. If the high risk is confirmed by the clinical examination, the clinician will re-evaluate the CTX and adjust the CTX regimen accordingly, in step 605. As indicated by dashed lines, the clinical examination may be omitted. If the prediction data instead designates risk class "SUSPECT", the clinician may decide (step 603) that the prediction method 210 should be operated on input data of extended scope (step 606). The extended input data may include further and/or other variables of VPD, CTD, or PD compared to the default input data used in step 600. Step 606 may include acquiring VPD by performing measurements in accordance with an extended protocol. The example in FIG. 6 presumes that step 606 generates the prediction data to designate either risk class "LOW" or risk class "HIGH". If the prediction data from step 606 designates risk class "HIGH", the clinician will re-evaluate the CTX (step 605). If the prediction data from step 606 instead designates risk class "LOW", the clinician may put the patient on routine monitoring for CIPN (step 602).

Some advantages are readily apparent from FIG. 6. For one, each of steps 600, 606 enable early detection of patients that are prone to develop CIPN. Thus, countermeasures may be taken at an early stage to mitigate or prevent CIPN. This may be particularly relevant for long-term CIPN, which may become chronic and lead to unnecessary suffering. Second, the separation of the patients in the intermediate risk class "SUSPECT" by step 603 will reduce the time spent by clinicians or physicians on potentially unnecessary re-evaluation of therapy. Instead, the need for re-evaluation is quantified by step 606, which may be performed in time efficient manner by less medically qualified staff.

FIG. 7 illustrates another use case. At a time point t = t1, input data 21 is acquired, and two prediction modules 300A, 300B are operated on the input data 21. One prediction module 300A (MOD1) is configured to determine the risk for short-term CIPN, and the other prediction module 300B (MOD2) is configured to determine the risk for long-term CIPN. MOD1 and MOD2 operate on input data 21, but it is understood that MOD1 and MOD2 may operate on at least partly different parts of the input data 21. MOD1 and MOD2 generate prediction data with one of three risk classes: "LOW", "HIGH" and "SUSPECT". MOD1 and MOD2 may be included in one prediction device. For example, the time point t = t1 may be before the initial session of CTX. A patient that is designated risk class "LOW" or "HIGH" by at least one of the modules may be managed as described for FIG. 6. However, if the patient is designated risk class "SUSPECT", the prediction device may output a recommendation for the clinician to perform a CIPN prediction also at a subsequent time point t = t2 during the CTX, for example before the next session. This recommendation is indicated by arrow 700 in FIG. 7. In the illustrated example, the CIPN prediction is performed at t = t2 by two other prediction modules 300C, 300D (MOD3, MOD4) which are operated on input data 21 acquired at t = t2. Like MOD1 and MOD2, MOD3 and MOD4 are configured to determine the risk for short-term CIPN and long-term CIPN, respectively, but MOD3 and MOD4 generate prediction data with one of two risk classes: "LOW" and "HIGH". Thereby, the separation of "SUSPECT" into "LOW" and "HIGH" (cf. step 607 in FIG. 6) is distributed in time across treatment sessions.

FIGS 8A-8C are ROC plots for three different examples of prediction modules that are configured to designate one of two predefined risk classes, "HIGH" and "LOW". The prediction modules are thus so-called binary classifier systems. The ROC plot is a well-known technique of illustrating the diagnostic ability of a binary classifier system as its discrimination threshold is varied. An ROC curve is created by plotting the true positive rate (TPR) against the false positive rate (FPR) at various threshold settings. TPR is also known as sensitivity. FPR is also known as probability of false alarm and may be calculated as (1 - specificity), where the specificity if the true-negative rate (TNR). An ROC curve may be used to evaluate the performance of a binary classifier system and also to set the value of the discrimination threshold. In FIGS 8A-8C, a diagonal dashed line represents the result of a random guess, and the farther the ROC curve is distanced to the left of the diagonal line, the better is the prediction. FIGS 8A-8C all indicate that the respective prediction module has good predictive ability. According to an example criterion, a method is deemed practically useful is the sum of the sensitivity and specificity is at least 1.5. This criterion is fulfilled for a large portion of the respective ROC curve 801-803 in FIGS 8A-8C. The underlying conditions and assumptions for the respective ROC plot have already been presented above and will not be repeated.

The structures and methods disclosed herein may be implemented by hardware or a combination of software and hardware. In some embodiments, the hardware comprises one or more software-controlled processors. FIG. 9 schematically depicts a prediction device 20, which comprises an input interface 20A and an output interface 20B. The interfaces 20A, 20B may be implemented by hardware or a combination of software and hardware and may operate in accordance with any standardized or proprietary protocol for wired or wireless data communication. The prediction device 20 further comprises processing circuitry 901 and computer memory 902. The processing circuitry 901 may include one or more of a CPU ("Central Processing Unit"), a DSP ("Digital Signal Processor"), a GPU ("Graphics Processing Unit"), a microprocessor, a microcontroller, an ASIC ("Application-Specific Integrated Circuit"), a combination of discrete analog and/or digital components, or some other programmable logical device, such as an FPGA ("Field Programmable Gate Array"). A control program 902A comprising computer instructions is stored in the memory 902 and executed by the processing circuitry 901 to implement logic that performs any of the methods, procedures, functions, operations, or steps described in the foregoing. The control program 902A may be supplied to the prediction device 20 on a computer-readable medium 910, which may be a tangible (non-transitory) product (e.g. magnetic medium, optical disk, read-only memory, flash memory, etc.) or a propagating signal. As indicated in FIG. 9, the memory 902 may also store control data 902B for use by the processing circuitry 901, such as the above-mentioned prediction model(s), weight factors of the prediction model(s), discrimination thresholds, limits, etc.

Further, while operations are depicted in the drawings in a particular order, this should not be understood as requiring that such operations be performed in the particular order shown or in sequential order, or that all illustrated operations be performed, to achieve desirable results. In certain circumstances, parallel processing may be advantageous.

## Claims

1. A prediction device, comprising circuitry (901) configured to predict a risk of chemotherapy-induced peripheral neuropathy, CIPN, in a test subject, said circuitry (901) being configured to:

   receive input data comprising perception data (301) that designates measured perception of vibrations at one or more predetermined locations on one or more limbs of the test subject, wherein the perception data (301) represents, for vibrations at each of one or more predefined frequencies, a vibration energy that causes the test subject to switch between perception and non-perception of the vibrations,
   operate at least one prediction model (32, 32') on the perception data (301) to determine at least one risk variable (331, 331'), which is indicative of a risk that the test subject will develop peripheral neuropathy as a result of chemotherapy, and
   generate, based on the at least one risk variable (331, 331'), prediction data (351) which is indicative of a predicted risk for CIPN,
   wherein at least one of the one or more predefined frequencies is below 60 Hz, and
   wherein the at least one risk variable (331, 331') comprises a risk variable which is indicative of the risk that the test subject will develop CIPN that persists at least six months after completion of the chemotherapy.

2. The prediction device of claim 1, wherein the one or more predefined frequencies comprises at least two different frequencies below 64 Hz.

3. The prediction device of claim 1 or 2, wherein said at least one of the one or more predefined frequencies is at or below 60 Hz, 50 Hz, 40 Hz, 35 Hz, 30 Hz, 25 Hz, 20 Hz, 15 Hz, or 10 Hz.

4. The prediction device of any preceding claim, wherein the perception data (301) comprises a plurality of perception values that differ by at least one of: predefined frequency, predetermined location, or limb of the test subject.

5. The prediction device of any preceding claim, wherein the chemotherapy comprises a time sequence of sessions (TS) with administration of at least one chemotherapeutic agent, and wherein the perception data (301) represents the measured perception of the vibrations by the test subject prior to at least one session in the time sequence of sessions (TS).

6. The prediction device of claim 5, wherein the perception data (301) represents the measured perception of the vibrations by the test subject prior to at least an initial session in the time sequence of sessions (TS).

7. The prediction device of any preceding claim, wherein the chemotherapy comprises administration of at least one chemotherapeutic agent in the group consisting of: platinum-containing chemotherapeutic agents, taxanes, immunomodulatory agents, vinca alkaloids, epothilones, and protease inhibitors.

8. The prediction device of any preceding claim, wherein the input data further comprises a set of parameter values (302, 303) representing the test subject and/or the chemotherapy, and wherein said circuitry (901) is configured to determine the at least one risk variable by operating the at least one prediction model (32, 32') on the perception data (301) and on the set of parameter values (302, 303).

9. The prediction device of claim 8, wherein the set of parameter values (302) is indicative of one or more of: an age of the test subject, a gender of the test subject, one or more physical characteristics of the test subject, a current temperature of the test subject, a health status of the test subject, a medication status of the test subject, and a medical history of the test subject.

10. The prediction device of claim 8 or 9, wherein the set of parameter values (303) is indicative of one or more of: a chemotherapy treatment history of the test subject, a chemotherapeutic agent administered in the chemotherapy, an accumulated dose of the chemotherapeutic agent administered during the chemotherapy, a method of administering the chemotherapeutic agent, and a schedule of the chemotherapy.

11. The prediction device of any preceding claim, wherein the circuitry (901) is further configured to: generate a plurality of variables based on the input data, wherein the at least one prediction model (32) is configured to determine the at least one risk variable (331) by combining the plurality of variables by use of a plurality of predetermined weight factors.

12. The prediction device of any preceding claim, wherein the prediction data (351) is indicative of one of at least three predefined risk classes (351A, 351B, 351C), wherein the at least three predefined risk classes comprise: a first risk class (351A) associated with a low risk, a second risk class (351B) associated with a high risk, and a third risk class (351C) intermediate the first and second risk classes.

13. The prediction device of any preceding claim, wherein the at least one risk variable comprises a first risk variable and a second risk variable (331, 331'), and wherein the circuitry (901) is further configured to: determine a first category based on the first risk variable (331), determine a second category based on the second risk variable (331'), and generate the prediction data (351) as a logical combination of the first category and the second category, and wherein the circuitry (901) is further configured to: operate a first prediction model (32) on the input data (301) to determine the first risk variable, and operate a second prediction model (32') on the input data (301) to determine the second risk variable, wherein the first risk variable is indicative of a low risk of developing CIPN, and the second risk variable is indicative of a high risk of developing CIPN.

14. The prediction device of any preceding claim, wherein said circuitry (901) is further configured to: evaluate (310) the input data in relation to a set of content requirements to determine an adequacy score, and selectively, based on the adequacy score, output (313) a request for further input data.

15. A computer-implemented prediction method, comprising:

>   receiving (211) input data comprising perception data that designates measured perception of vibrations at one or more predetermined locations on one or more limbs of a test subject, wherein the perception data represents, for vibrations at each of one or more predefined frequencies, a vibration energy that causes the test subject to switch between perception and non-perception of the vibrations;
>   operating (214) a prediction model on the perception data to determine at least one risk variable, which is indicative of a risk that the test subject will develop peripheral neuropathy as a result of chemotherapy; and
>   generating (215), based on the at least one risk variable, prediction data which is indicative of a predicted risk for CIPN,
>   wherein at least one of the one or more predefined frequencies is below 60 Hz, and
>   wherein the at least one risk variable comprises a risk variable which is indicative of the risk that the test subject will develop chemotherapy-induced peripheral neuropathy that persists at least six months after completion of the chemotherapy.

**Patentansprüche**

1. Vorhersagevorrichtung, die eine Schaltungsanordnung (901) umfasst, die dazu ausgelegt ist, ein Risiko einer Chemotherapie-induzierten peripheren Neuropathie, CIPN, in einem Testsubjekt vorherzusagen, wobei die Schaltungsanordnung (901) ausgelegt ist zum:

>   Empfangen von Eingabedaten, die Wahrnehmungsdaten (301) umfassen, die gemessene Wahrnehmung von Vibrationen an einem oder mehreren vorbestimmten Orten an einer oder mehreren Extremitäten des Testsubjekts bezeichnen, wobei die Wahrnehmungsdaten (301) für Vibrationen bei jeder von einer oder mehreren vordefinierten Frequenzen eine Vibrationsenergie repräsentieren, die bewirkt, dass das Testsubjekt zwischen Wahrnehmung und Nichtwahrnehmung der Vibrationen wechselt,
>   Betreiben mindestens eines Vorhersagemodells (32, 32') an den Wahrnehmungsdaten (301), um mindestens eine Risikovariable (331, 331') zu bestimmen, die ein Risiko angibt, dass das Testsubjekt als Ergebnis einer Chemotherapie periphere Neuropathie entwickeln wird, und Generieren von Vorhersagedaten (351), die ein vorhergesagtes Risiko für CIPN angeben, basierend auf der mindestens einen Risikovariable (331, 331'),
>   wobei mindestens eine der einen oder mehreren vordefinierten Frequenzen unter 60 Hz liegt, und
>   wobei die mindestens eine Risikovariable (331, 331') eine Risikovariable umfasst, die das Risiko angibt, dass das Testsubjekt CIPN entwickeln wird, die mindestens sechs Monate nach Abschluss der Chemotherapie persistiert.

2. Vorhersagevorrichtung nach Anspruch 1, wobei die eine oder mehreren vordefinierten Frequenzen mindestens zwei unterschiedliche Frequenzen unter 64 Hz umfassen.

3. Vorhersagevorrichtung nach Anspruch 1 oder 2, wobei die mindestens eine der einen oder mehreren vordefinierten Frequenzen bei oder unter 60 Hz, 50 Hz, 40 Hz, 35 Hz, 30 Hz, 25 Hz, 20 Hz, 15 Hz oder 10 Hz liegt.

4. Vorhersagevorrichtung nach einem der vorhergehenden Ansprüche, wobei die Wahrnehmungsdaten (301) eine Vielzahl von Wahrnehmungswerten umfassen, die sich um mindestens eines von vordefinierter Frequenz, vorbestimmter Position oder Extremität des Testsubjekts unterscheiden.

5. Vorhersagevorrichtung nach einem der vorhergehenden Ansprüche, wobei die Chemotherapie eine Zeitsequenz von Sitzungen (TS) mit Verabreichung mindestens eines Chemotherapeutikums umfasst, und wobei die Wahrnehmungsdaten (301) die gemessene Wahrnehmung der Vibrationen durch das Testsubjekt vor mindestens einer Sitzung in der Zeitsequenz von Sitzungen (TS) repräsentieren.

6. Vorhersagevorrichtung nach Anspruch 5, wobei die Wahrnehmungsdaten (301) die gemessene Wahrnehmung der Vibrationen durch das Testsubjekt vor mindestens einer anfänglichen Sitzung in der Zeitsequenz von Sitzungen (TS) repräsentieren.

7. Vorhersagevorrichtung nach einem der vorhergehenden Ansprüche, wobei die Chemotherapie Verabreichung mindestens eines Chemotherapeutikums aus der Gruppe bestehend aus platinhaltigen Chemotherapeutika, Taxanen, immunmodulatorischen Mitteln, Vinca-Alkaloiden, Epothilonen, und Proteaseinhibitoren umfasst.

8. Vorhersagevorrichtung nach einem der vorhergehenden Ansprüche, wobei die Eingabedaten ferner einen Satz von Parameterwerten (302, 303) umfassen, die das Testsubjekt und/oder die Chemotherapie repräsentieren, und wobei die Schaltungsanordnung (901) dazu ausgelegt ist, die mindestens eine Risikovariable durch Betreiben des mindestens einen Vorhersagemodells (32, 32') an den Wahrnehmungsdaten (301) und an dem Satz von Parameterwerten (302, 303) zu bestimmen.

9. Vorhersagevorrichtung nach Anspruch 8, wobei der Satz von Parameterwerten (302) eines oder mehrere von einem Alter des Testsubjekts, einem Geschlecht des Testsubjekts, einer oder mehreren physischen Eigenschaften des Testsubjekts, einer aktuellen Temperatur des Testsubjekts, einem Gesundheitsstatus des Testsubjekts, einem Medikamentenstatus des Testsubjekts und einer medizinischen Vorgeschichte des Testsubjekts angibt.

10. Vorhersagevorrichtung nach Anspruch 8 oder 9, wobei der Satz von Parameterwerten (303) eines oder mehrere von einer Chemotherapiebehandlungshistorie des Testsubjekts, einem in der Chemotherapie verabreichten Chemotherapeutikum, einer akkumulierten Dosis des während der Chemotherapie verabreichten Chemotherapeutikums, einem Verfahren zur Verabreichung des Chemotherapeutikums und einem Zeitplan der Chemotherapie angibt.

11. Vorhersagevorrichtung nach einem der vorhergehenden Ansprüche, wobei die Schaltungsanordnung (901) ferner ausgelegt ist zum Generieren einer Vielzahl von Variablen basierend auf den Eingabedaten, wobei das mindestens eine Vorhersagemodell (32) ausgelegt ist zum Bestimmen der mindestens einen Risikovariable (331) durch Kombinieren der Vielzahl von Variablen unter Verwendung einer Vielzahl von vorbestimmten Gewichtungsfaktoren.

12. Vorhersagevorrichtung nach einem der vorhergehenden Ansprüche, wobei die Vorhersagedaten (351) eine von mindestens drei vordefinierten Risikoklassen (351A, 351B, 351C) angeben, wobei die mindestens drei vordefinierten Risikoklassen eine erste Risikoklasse (351A), die mit einem niedrigen Risiko assoziiert ist, eine zweite Risikoklasse (351B), die mit einem hohen Risiko assoziiert ist, und eine dritte Risikoklasse (351C), die zwischen der ersten und der zweiten Risikoklasse liegt, umfassen.

13. Vorhersagevorrichtung nach einem der vorhergehenden Ansprüche, wobei die mindestens eine Risikovariable eine erste Risikovariable und eine zweite Risikovariable (331, 331') umfasst, und wobei die Schaltungsanordnung (901) ferner ausgelegt ist zum Bestimmen einer ersten Kategorie basierend auf der ersten Risikovariable (331), Bestimmen einer zweiten Kategorie basierend auf der zweiten Risikovariable (331') und Generieren der Vorhersagedaten (351) als eine logische Kombination der ersten Kategorie und der zweiten Kategorie, und wobei die Schaltungsanordnung (901) ferner ausgelegt ist zum Betreiben eines ersten Vorhersagemodells (32) an den Eingabedaten (301), um die erste Risikovariable zu bestimmen, und Betreiben eines zweiten Vorhersagemodells (32') an den Eingabedaten (301), um die zweite Risikovariable zu bestimmen, wobei die erste Risikovariable ein geringes Risiko des Entwickelns von CIPN angibt und die zweite Risikovariable ein hohes Risiko des Entwickelns von CIPN angibt.

14. Vorhersagevorrichtung nach einem der vorhergehenden Ansprüche, wobei die Schaltungsanordnung (901) ferner ausgelegt ist zum Evaluieren (310) der Eingabedaten in Bezug auf einen Satz von Inhaltsanforderungen, um einen Angemessenheits-Score zu bestimmen, und selektiven Ausgeben (313) einer Anforderung für weitere Eingabedaten basierend auf dem Angemessenheits-Score.

15. Computerimplementiertes Vorhersageverfahren, umfassend:

Empfangen (211) von Eingabedaten, die Wahrnehmungsdaten umfassen, die gemessene Wahrnehmung von Vibrationen an einem oder mehreren vorbestimmten Orten an einer oder mehreren Extremitäten eines Testsubjekts bezeichnen, wobei die Wahrnehmungsdaten für Vibrationen bei jeder von einer oder mehreren vordefinierten Frequenzen eine Vibrationsenergie repräsentieren, die bewirkt, dass das Testsubjekt zwischen Wahrnehmung und Nichtwahrnehmung der Vibrationen wechselt;
Betreiben (214) eines Vorhersagemodells an den Wahrnehmungsdaten, um mindestens eine Risikovariable zu bestimmen, die ein Risiko angibt, dass das Testsubjekt als Ergebnis einer Chemotherapie periphere Neuropathie entwickeln wird; und

Generieren (215) von Vorhersagedaten, die ein vorhergesagtes Risiko für CIPN angeben, basierend auf der mindestens einen Risikovariable,

wobei mindestens eine der einen oder mehreren vordefinierten Frequenzen unter 60 Hz liegt, und

wobei die mindestens eine Risikovariable eine Risikovariable umfasst, die das Risiko angibt, dass das Testsubjekt Chemotherapie-induzierte periphere Neuropathie entwickeln wird, die mindestens sechs Monate nach Abschluss der Chemotherapie persistiert.

## Revendications

1. Dispositif de prédiction, comprenant un montage de circuits (901) configuré pour prédire un risque de neuropathie périphérique chimio-induite, NPCI, chez un sujet testé, ledit montage de circuits (901) étant configuré pour :

recevoir des données d'entrée comprenant des données de perception (301) qui désignent une perception mesurée de vibrations à un ou plusieurs emplacements prédéterminés sur un ou plusieurs membres du sujet testé, dans lequel les données de perception (301) représentent, pour des vibrations à chacune d'une ou de plusieurs fréquences prédéfinies, une énergie vibratoire qui amène le sujet testé à basculer entre la perception et la non-perception des vibrations,

mettre en œuvre au moins un modèle de prédiction (32, 32') sur les données de perception (301) afin de déterminer au moins une variable de risque (331, 331'), qui indique un risque que le sujet testé développe une neuropathie périphérique suite en résultat à une chimiothérapie, et

générer, sur la base de l'au moins une variable de risque (331, 331'), des données de prédiction (351) qui indiquent un risque prédit de NPCI,

dans lequel au moins une des une ou plusieurs fréquences prédéfinies est inférieure à 60 Hz, et dans lequel l'au moins une variable de risque (331, 331') comprend une variable de risque qui indique le risque que le sujet testé développe une NPCI qui persiste au moins six mois après la fin de la chimiothérapie.

2. Dispositif de prédiction selon la revendication 1, dans lequel les une ou plusieurs fréquences prédéfinies comprennent au moins deux fréquences différentes inférieures à 64 Hz.

3. Dispositif de prédiction selon la revendication 1 ou 2, dans lequel ladite au moins une des une ou plusieurs fréquences prédéfinies est égale ou inférieure à 60 Hz, 50 Hz, 40 Hz, 35 Hz, 30 Hz, 25 Hz, 20 Hz, 15 Hz ou 10 Hz.

4. Dispositif de prédiction selon une quelconque revendication précédente, dans lequel les données de perception (301) comprennent une pluralité de valeurs de perception qui diffèrent d'au moins l'un parmi : une fréquence prédéfinie, un emplacement prédéterminé ou un membre du sujet testé.

5. Dispositif de prédiction selon une quelconque revendication précédente, dans lequel la chimiothérapie comprend une séquence temporelle de séances (TS) avec administration d'au moins un agent chimiothérapeutique, et dans lequel les données de perception (301) représentent la perception mesurée des vibrations par le sujet testé avant au moins une séance dans la séquence temporelle de séances (TS).

6. Dispositif de prédiction selon la revendication 5, dans lequel les données de perception (301) représentent la perception mesurée des vibrations par le sujet testé avant au moins une session initiale dans la séquence temporelle de sessions (TS).

7. Dispositif de prédiction selon une quelconque revendication précédente, dans lequel la chimiothérapie comprend l'administration d'au moins un agent chimiothérapeutique dans le groupe constitué par : des agents chimiothérapeutiques contenant du platine, des taxanes, des agents immunomodulateurs, des vinca-alcaloïdes, des épothilones, et des inhibiteurs de la protéase.

8. Dispositif de prédiction selon une quelconque revendication précédente, dans lequel les données d'entrée comprennent en outre un ensemble de valeurs de paramètres (302, 303) représentant le sujet testé et/ou la chimiothérapie, et dans lequel ledit montage de circuits (901) est configuré pour déterminer l'au moins une variable de risque en faisant fonctionner l'au moins un modèle de prédiction (32, 32') sur les données de perception (301) et sur l'ensemble de valeurs de paramètres (302, 303).

9. Dispositif de prédiction selon la revendication 8, dans lequel l'ensemble de valeurs de paramètres (302) indique un ou plusieurs parmi : l'âge du sujet testé, le sexe du sujet testé, une ou plusieurs caractéristiques physiques du sujet testé, la température actuelle du sujet testé, l'état de santé du sujet testé, l'état de médication du sujet testé, et les antécédents médicaux du sujet testé.

10. Dispositif de prédiction selon la revendication 8 ou 9, dans lequel l'ensemble de valeurs de paramètres (303) indique un ou plusieurs parmi : un historique de traitements chimiothérapeutiques du sujet testé, un agent chimiothérapeutique administré dans la chimiothérapie, une dose accumulée de l'agent chimiothérapeutique administré pendant la chimiothérapie, un procédé d'administration de l'agent chimiothérapeutique, et un calendrier de la chimiothérapie.

11. Dispositif de prédiction selon une quelconque revendication précédente, dans lequel le montage de circuits (901) est en outre configuré pour : générer une pluralité de variables sur la base des données d'entrée, dans lequel l'au moins un modèle de prédiction (32) est configuré pour déterminer l'au moins une variable de risque (331) en combinant la pluralité de variables par l'utilisation d'une pluralité de facteurs de pondération prédéterminés.

12. Dispositif de prédiction selon une quelconque revendication précédente, dans lequel les données de prédiction (351) indiquant l'une d'au moins trois classes de risque prédéfinies (351A, 351B, 351C), dans lequel les au moins trois classes de risque prédéfinies comprennent : une première classe de risque (351A) associée à un risque faible, une deuxième classe de risque (351B) associée à un risque élevé, et une troisième classe de risque (351C) entre les première et deuxième classes de risque.

13. Dispositif de prédiction selon une quelconque revendication précédente, dans lequel l'au moins une variable de risque comprend une première variable de risque et une seconde variable de risque (331, 331'), et dans lequel le montage de circuits (901) est en outre configuré pour : déterminer une première catégorie sur la base de la première variable de risque (331), déterminer une seconde catégorie sur la base de la seconde variable de risque (331'), et générer les données de prédiction (351) en tant que combinaison logique de la première catégorie et de la seconde catégorie, et dans lequel le montage de circuits (901) est en outre configuré pour : faire fonctionner un premier modèle de prédiction (32) sur les données d'entrée (301) pour déterminer la première variable de risque, et faire fonctionner un second modèle de prédiction (32') sur les données d'entrée (301) pour déterminer la seconde variable de risque, dans lequel la première variable de risque indique un faible risque de développer une NPCI, et la seconde variable de risque indique un risque élevé de développer une NPCI.

14. Dispositif de prédiction selon une quelconque revendication précédente, dans lequel ledit montage de circuits (901) est en outre configuré pour : évaluer (310) les données d'entrée en relation avec un ensemble d'exigences de contenu pour déterminer un score d'adéquation, et sélectivement, sur la base du score d'adéquation, sortir (313) une demande pour d'autres données d'entrée.

15. Procédé de prédiction mis en œuvre par ordinateur, comprenant :

la réception (211) de données d'entrée comprenant des données de perception qui désignent une perception mesurée de vibrations à un ou plusieurs emplacements prédéterminés sur un ou plusieurs membres du sujet testé, dans lequel les données de perception représentent, pour des vibrations à chacune d'une ou de plusieurs fréquences prédéfinies, une énergie vibratoire qui amène le sujet testé à basculer entre la perception et la non-perception des vibrations,
la mise en œuvre (214) d'un modèle de prédiction sur les données de perception afin de déterminer au moins une variable de risque, qui indique un risque que le sujet testé développe une neuropathie périphérique en résultat à une chimiothérapie ; et
la génération (215), sur la base de l'au moins une variable de risque, de données de prédiction qui indiquent un risque prédit de NPCI,
dans lequel au moins une des une ou plusieurs fréquences prédéfinies est inférieure à 60 Hz, et dans lequel l'au moins une variable de risque comprend une variable de risque qui indique le risque que le sujet testé développe une neuropathie périphérique chimio-induite qui persiste au moins six mois après la fin de la chimiothérapie.

FIG. 1A

FIG. 1B

FIG. 1C

200 — DETERMINE PERCEPTION DATA

201 — INDUCE VIBRATIONS AT ONE OR MORE PREDEFINED FREQUENCIES IN ONE OR MORE PREDETERMINED LOCATIONS ON ONE OR MORE LIMBS OF TEST SUBJECT

202 — RECEIVE FEEDBACK FROM TEST SUBJECT

203 — COMPUTE, BASED ON FEEDBACK, VPT(S) REPRESENTING VIBRATION ENERGY FOR SWITCH BETWEEN PERCEPTION AND NON-PERCEPTION OF VIBRATIONS

FIG. 1D

20

20A

21 → 300 → 22

20B

FIG. 2A

210 — CIPN PREDICTION

211 — RECEIVE VPD FOR ONE OR MORE FREQUENCIES BELOW 64 HZ (LF-VPD)

212 — RECEIVE PATIENT DATA (PD) REPRESENTING TEST SUBJECT

213 — RECEIVE THERAPY DATA (CTD) REPRESENTING CHEMOTHERAPY

214 — OPERATE PREDICTION MODEL ON VPD, AND OPTIONALLY PD AND/OR CTD, TO DETERMINE ONE OR MORE RISK VARIABLES FOR DEVELOPMENT OF CIPN

215 — GENERATE PREDICTION DATA BASED ON RISK VARIABLE(S)

FIG. 2B

FIG. 2C

FIG. 3A

FIG. 3B

FIG. 4A

FIG. 4B

FIG. 4C

FIG. 5

FIG. 6

t = t1                                    t = t2

FIG. 7

FIG. 8A

FIG. 8B

FIG. 8C

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006046901 A

**Non-patent literature cited in the description**

- Quantitative Sensory Testing at Baseline and During Cycle 1 Oxaliplatin Infusion Detects Subclinical Peripheral Neuropathy and Predicts Clinically Overt Chronic Neuropathy in Gastrointestinal Malignancies. **REDDY et al.** Clinical Colorectal Cancer. 2016, vol. 15, 37-46